# EUROPÄISCHE PATENTSCHRIFT

(11) **EP 2 663 366 B1**
(45) Veröffentlichungstag und Bekanntmachung des Hinweises auf die Patenterteilung: **05.08.2020**
(21) Anmeldenummer: 12701665.7
(22) Anmeldetag: 06.01.2012
(51) Int. Cl.: A61K 31/198, A61P 15/06, A61P 43/00, A61P 3/02, A23L 33/00, A23L 33/175

(54) **AMINOSÄUREZUSAMMENSETZUNG UND DEREN VERWENDUNG ZUR BEHANDLUNG VON INTRAUTERINER FETALER WACHSTUMSRESTRIKTION (IUGR) ODER ZUR PARENTERALEN ERNÄHRUNG VON EXTREM FRÜHGEBORENEN**
AMINO ACID COMPOUND AND USE OF SAME FOR TREATING INTRAUTERINE GROWTH RESTRICTION (IUGR) AND FOR PARENTERAL FEEDING OF EXTREMELY PREMATURE INFANTS
COMPOSITION D'ACIDE AMINÉ ET SON UTILISATION POUR LE TRAITEMENT DE LA RESTRICTION DE CROISSANCE FÝTALE (IUGR) ET POUR L'ALIMENTATION PARENTÉRALE DE GRANDS PRÉMATURÉS

(30) Priorität: 13.01.2011 EP 11000211
(43) Veröffentlichungstag der Anmeldung: 20.11.2013
(73) Patentinhaber: Tchirikov, Michael, 06126 Halle (Saale) (DE)
(72) Erfinder: Tchirikov, Michael, 06126 Halle (Saale) (DE)
(74) Vertreter: Patentanwälte Dr. Keller, Schwertfeger Partnerschaft mbB
(86) Internationale Anmeldenummer: PCT/EP2012/000045
(87) Internationale Veröffentlichungsnummer: WO 2012/095281

(56) Entgegenhaltungen:
- WO-A1-2007/016615
- WO-A1-2007/121807
- TCHIRIKOV M ET AL: "Treatment of Growth-Restricted Human Fetuses with Amino Acids and Glucose Supplementation through a Chronic Fetal Intravascular Perinatal Port System", EUROPEAN SURGICAL RESEARCH, Bd. 45, Nr. 1, 2010, Seiten 45-49, XP002638384, ISSN: 0014-312X in der Anmeldung erwähnt
- Fresenius Kabi New Zealand Limited: "Vaminolact", Medicine Datasheet , 31. März 2010 (2010-03-31), Seiten 1-5, XP002638385, Gefunden im Internet: URL:http://www.medsafe.govt.nz/profs/datas heet/v/Vaminolactinf.pdf [gefunden am 2011-05-23]
- RONZONI STEFANIA ET AL: "The effect of a maternal infusion of amino acids on umbilical uptake in pregnancies complicated by intrauterine growth restriction", AMERICAN JOURNAL OF OBSTETRICS AND GYNECOLOGY, Bd. 187, Nr. 3, September 2002 (2002-09), Seiten 741-746, XP002638386, ISSN: 0002-9378
- Anonymous: "FreAmine III", drugs.com , Oktober 2010 (2010-10), XP002638387, Gefunden im Internet: URL:http://www.drugs.com/pro/10-percent-fr eamine-iii.html [gefunden am 2011-03-23]
- BROWN LAURA D ET AL: "Maternal amino acid supplementation for intrauterine growth restriction.", FRONTIERS IN BIOSCIENCE (SCHOLAR EDITION), Bd. 3, 1. Januar 2011 (2011-01-01), Seiten 428-444, XP009148696, ISSN: 1945-0524
- CLARK REESE H ET AL: "Effects of two different doses of amino acid supplementation on growth and blood amino acid levels in premature neonates admitted to the neonatal intensive care unit: A randomized, controlled trial", PEDIATRICS, Bd. 120, Nr. 6, Dezember 2007 (2007-12), Seiten 1286-1296, XP002638388,
- ADAMKIN D H ET AL: "Comparison of two neonatal intravenous amino acid formulations in preterm infants: a multicenter study.", JOURNAL OF PERINATOLOGY : OFFICIAL JOURNAL OF THE CALIFORNIA PERINATAL ASSOCIATION, Bd. 11, Nr. 4, Dezember 1991 (1991-12), Seiten 375-382, XP009148695, ISSN: 0743-8346
- VALENTINE C J ET AL: "Early amino-acid administration improves preterm infant weight", JOURNAL OF PERINATOLOGY, Bd. 29, Nr. 6, 2009, Seiten 428-432, XP002638390, ISSN: 0743-8346, DOI: 10.1038/JP.2009.51
- NATIONAL INSTITUTE OF CHILD HEALTH AND HUMAN DEVELOPMENT NEONATAL RESEARCH NETWORK POINDEXTER ET AL: "Early provision of parenteral amino acids in extremely low birth weight infants: Relation to growth and neurodevelopmental outcome", JOURNAL OF PEDIATRICS, Bd. 148, Nr. 3, 1. März 2006 (2006-03-01), Seiten 300-305.E1, XP005674533, MOSBY-YEAR BOOK, ST. LOUIS, MO, US ISSN: 0022-3476, DOI: 10.1016/J.JPEDS.2005.10.038
- TE BRAAKE ET AL: "Amino Acid Administration to Premature Infants Directly After Birth", JOURNAL OF PEDIATRICS, Bd. 147, Nr. 4, 1. Oktober 2005 (2005-10-01), Seiten 457-461, XP005111953, MOSBY-YEAR BOOK, ST. LOUIS, MO, US ISSN: 0022-3476, DOI: 10.1016/J.JPEDS.2005.05.038

## Beschreibung

Aminosäurezusammensetzung und deren Verwendung zur Behandlung von intrauteriner fetaler Wachstumsrestriktion (IUGR) oder zur parenteralen Ernährung von extrem Frühgeborenen.

### Beschreibung:

Die vorliegende Erfindung betrifft eine Aminosäurezusammensetzung und deren Verwendung zur Behandlung von intrauteriner fetaler Wachstumsrestriktion (IUGR) oder zur parenteralen Ernährung von extrem Frühgeborenen in der 24. bis 34. Schwangerschaftswoche.

### Gebiet der Erfindung:

Die Erfindung liegt auf medizinischem und pharmakologischem Gebiet.

### Stand der Technik:

Eine intrauterine Wachstumsretardierung (Restriktion) des Fötus (Intrauterine Growth Restriction (IUGR)) wird hauptsächlich durch eine Plazentainsuffizienz der Mutter hervorgerufen und stellt eine schwerwiegende Komplikation während der Schwangerschaft dar. IUGR ist eine der Hauptursachen für Frühgeburt, welche wiederum zum Tode des Frühgeborenen führen kann (Goldenberg et al., Epidemology and causes of preterm birth, Lancet 2008;371:75-84). Zu den anderen Ursachen der IUGR zählen genetische Vorbelastungen, mütterliche Erkrankungen oder starker Drogenkonsum der Mutter. Eine intrauterine Wachstumsrestriktion wird während der Schwangerschaft durch eine Ultraschallkontrolle festgestellt.

Die fetale Wachstumsretardation kommt in ungefähr in 3 bis 5 % aller Schwangerschaften vor. Dabei stellt die chronische Hypoxia in IUGR-Föten die Hauptursache für die neonatalen Komplikationen dar (Salihu et al., Is small for gestational age a marker of future fetal survival in utero? Obstet Gynecol 2006;107:851-856).

In vielen Fällen erhöht sich die Sterberate beim Krankheitsverlauf bei IUGR-Föten um das 3- bis 10-fache. Etwa 25 bis 40 % aller absterbenden intrauterinen Föten sind IUGR-Föten (Salihu et al., Is small for gestational age a marker of future fetal survival in utero? Obstet Gynecol 2006; 107:851-856).

Die fetale Wachstumsretardation während der Schwangerschaft verschlechtert die Entwicklung des Neugeborenen und des Kindes beträchtlich (Pryor J. et al., Development and behaviour in adolescents born small-for-gestational-age. J Paediatr Child Health 1995;31:403-407). Kinder mit IUGR leiden an depressiven und kognitiven Krankheiten (Räikkönen et al., Depression in young adults with very low birth weight; the Helsinki study of very-low-birth-weight adults. Arch Gen Psychiatry 2008; 65:290-296). Die IUGR-Situation während der Schwangerschaft führt zu Veränderungen beim fetalen Programmablauf ("Barker-Hypothese"). Ferner wird die fetale Wachstumsretardation auch als Ursache für die Entwicklung von metabolischem Syndrom sowie für ein erhöhtes Risiko für die Entwicklung von koronaren Herzerkrankungen, Infarkten und Diabetes mellitus Typ 2 angesehen (Barker DJ et al., The fetal origins of adult hypertension, J Hypertens Suppl 1992;10:39-44; Fetal nutrition and cardiovascular disease in adult life. Lancet 1993;341:938-941; Fetal origins of coronary heart disease. BMJ 1995;311:171-174; In utero programming of chronic disease. Clin Sci 1998;95:115-128; Fetal programming of coronary heart disease, Trends Endocrinol Metab 2002;13:364-368).

Aufgrund des aktiven Transports von Aminosäuren von der Mutter zum Fötus ist die Konzentration der Aminosäuren im Nabelschnurblut des Fötus normalerweise um das 1,1- bis 3-fache höher als die Aminosäurenkonzentration des Blutplasmas der Mutter. Bei der IUGR ist der Aminosäurentransport durch die Plazenta reduziert, für einige Aminosäuren sogar ganz besonders (Ronzoni et al., The effect of a maternal infusion of amino acids on umbilical uptake in pregnancies complicated by intrauterine growth restriction. Am J Obstet Gynecol 2002; 187:741-6).

Ferner wurde festgestellt, dass auch der aktive Glucosetransport durch die Mikrovilli-Membran von Synytiotrophoblasten in IUGR-Föten signifikant vermindert ist (Jansson et al., Glucose transport and system A activity in syncytiotrophoblast microvillous and basal plasma membranes in intrauterine growth restriction. Placenta 2002;23:392-399).

Nach der Diagnose von IUGR sind die Optionen für eine Behandlung nur sehr beschränkt (Van Kamp et al., Complication of intrauterine intravascular transfusion for fetal anemia due to maternal red-cell alloimmunization. Am J Obstet Gynecol 2005;192:171-177). Die Patienten mit einer IUGR werden frühzeitig per Sectio entbunden, um einen intrauterinen Fruchttod durch die Mangelversorgung infolge einer Plazentainsuffizienz zu vermeiden. Es gibt keine nachgewiesene kausale Methode für die Behandlung der Plazentainsuffizienz, da eine "insuffiziente" Plazenta in der Frühschwangerschaft mit der folgenden fetalen Wachstumsretardierung am Ende des II. Trimenoms entsteht. Eine kontinuierliche Aminosäuren- und Glukose-Infusion in der Nabelschnurvene über ein subkutan implantiertes Portsystem konnte in Studien das fetale Wachstum verbessern und die Schwangerschaft um mehrere Wochen prolongieren (M. Tchirikov et al, treatment of growth-restricted human fetuses with amino acids and glucose supplementation through a chronic fetal intravascular perinatal port system, Case Report Eur Surg Res 2010;45:45-49).

Auch stehen nur begrenzte Therapiemöglichkeiten nach der extremen Frühgeburt zur Verfügung. In Betracht kommt eine Behandlung mit einer Aminosäurenlösung, welche die Versorgung der Frühgeborenen sicherstellen soll, wobei die Zusammensetzung der Aminosäuren, die der mütterlichen Milch ähnlich ist, sich deutlich von der im fetalen Plasma in utero unterscheidet. Das hat sehr wahrscheinlich negative Folgen für den Frühgeborenen (zum Beispiel veränderte Hirnentwicklung und Myelinisierung, signifikante Reduzierung des IQs, besondere mit der IUGR in der Anamnese). Zurzeit werden diese Komplikationen nur durch die Frühgeburt erklärt, wobei eine falsche parenterale Ernährung mit Aminosäuren, die auf der Milch-Basis basiert, selten in Betracht gezogen wird.

Aminosäurezusammensetzungen als solche sind generell für unterschiedliche Anwendungsgebiete im Stand der Technik hinreichend bekannt (man vergleiche beispielsweise die DE 698 31 609 T2, DE 693 32 433 A1, DE 699 33 738 T2, EP 2 116 238 A1, WO 2007/121807 A1, WO 2007/016615 A1).

Verschiedene Aminosäurezusammensetzungen zur Anwendung bei wachstumsretardierten Föten oder Neugeborenen sind bekannt. Eine Studie zeigt, dass eine intravenöse Aminosäurengabe an die Mutter bei wachstumsretardierten Föten zu einer signifikanten Dysbalancierung der Aminosäurenkomzentration bei Föten führt (Ronzoni et al., The effect of a maternal infusion on amino acids on umbilical uptake in pregnancies complicated by intrauterine growth restriction, Am J Obstet Gynecol, 2002). Die Konzentration von Threonin, Lysin, Histidin, Prolin und Alanin ist in dieser Studie im umbilikalen Blut nicht angestiegen, darunter auch drei essentielle Aminosäuren wie Lysin, Hystidin und Threonin. Dies deutet darauf hin, dass eine IUGR-bedingte Dysbalancierung der Aminosäurenkonzentration durch eine indirekte Aminosäurengabe an die Mutter verschlechtert wird. Ein Behandlungserfolg stellt sich hierbei nicht ein oder ist rein zufällig.

Eine weitere Studie zeigt, dass die Frühgeborene eine intravenöse Therapie mit Aminosäuren in einer Konzentration von 2.4 g/kg Körpergewicht pro Tag vertragen. Die durchgeführte Infusion hat die Konzentration der Aminosäuren bei den Frühgeborenen jedoch signifikant dysbalanciert (Braake FWJ et al., Amino acid administration to premature infants directly after birth, J Pediatr, 2005).

Ferner wurde eine frühere Aminosäurengabe mit einer späteren Gabe verglichen (Poindexter BB et al., Early provision amino acids in extremely low birth weight infants: Relation to growth an neurodevelopmental outcome, J Pediatrics, 2006) . Eine frühere Aminosäurengabe scheint für das Wachstum des Gehirnes eine positive Rolle zu haben. Die Konzentration der Aminosäuren wurde in der Studie nicht untersucht.

Auch andere, ähnliche Studien zeigten leider nur sehr unzureichende Ergebnisse für die Behandlung wachstumsretardierter Föten oder extrem Frühgeborener mit Aminosäurenenthaltenen Zusammensetzungen (Valentine CJ et al., Early amino acid administration improves preterm infant weight, J Perinat, 2009; Adamkin DH et al., Comparison of two neonatal intravenous amino acid formulations in preterm infants: a multicenter study, J Perinat, 1991; Clark RH et al., Effects oft two different doses of amino acid supplementation on growth and blood amino acid levels in premature neonates admitted to the neonatal intensive care unit: A randomised controlled rat, Pediatrics, 2007).

Schließlich wurde kürzlich festgestellt, dass eine mütterliche Aminosäurengabe für die Behandlung der intrauterinen Wachstumsretardierung das neonatale Outcome sogar verschlechtern könnte (Brown LD et al., Maternal amino acid suplementation for intrauterine growth restriction, Frontiers in Bioscience, 2011).

Im dem für die vorliegende Anmeldung nächstliegenden Stand der Technik wurde eine kommerziell erhältliche Aminosäurezusammensetzung (Vaminolact®) zur Behandlung von IUGR-Patienten über ein perinatales Port-System eingesetzt (M. Tchirikov et al., treatment of growth-restricted human fetuses with amino acids and glucose supplementation through a chronic fetal intravascular perinatal port system, Case Report EurSurg Res 2010;45:45-49). Die Studie zeigt allerdings nur den Ernährungseffekt der Behandlung. Insbesondere wird ein intravaskuläres Ergänzungssystem beschrieben, um Nährstoffe an einem IUGR-Fötus zur Verfügung zu stellen, um das fetale Wachstum zu verbessern und eine Verlängerung der Schwangerschaft sicherzustellen, da von IUGR betroffene Föten im Uterus nicht selbst die Fähigkeit besitzen, heranzuwachsen. Eine langfristige Ergänzung mit Nährstoffen über das perinatale Port-System durch transkutane Verabreichung von Aminosäuren und Glukose in die Nabelvene unter Verwendung des Port-Systems führte zu einer Erhöhung des fetalen Körpergewichtes und hatte das Potential zur Schwangerschaftsprolongation. Die Messung der Aminosäurenkonzentration in Plasma erfolgte nach der Geburt und eine Woche nach dem Absetzen der Infusion. Das maximale gegebene Volumen der Lösung mit 10% Glucose war auf 10% des fetalen Blutvolumens beschränkt. Damit war die tatsächliche Konzentration der Aminosäuren relativ klein und dadurch wie bei den Studien oben keine Verbesserung erreicht.

Daher gilt es, die Aminosäurezusammensetzung per se zu optimieren, um die Wirkung und somit Behandlungserfolg bei IUGR oder extrem Frühgeborenen signifikant zu verbessern.

Vor diesem Hintergrund ist es Aufgabe der vorliegenden Erfindung, eine verbesserte Zusammensetzung von Aminosäuren für die Verwendung zur Behandlung einer fetalen Wachstumsrestriktion (IUGR) oder zur parenteralen Ernährung von extrem Frühgeborenen in der 24. bis 34. Schwangerschaftswoche bereitzustellen, um dadurch eine intrauterine Wachstumsretardation des Fötus oder die Probleme bei extrem Frühgeborenen und die damit einhergehenden Folgen zu vermindern oder zu verhindern.

Diese Aufgabe wird durch eine Aminosäurezusammensetzung gemäß Anspruch 1 gelöst.

### Zusammenfassung der Erfindung:

Die vorliegende Erfindung betrifft eine Zusammensetzung für die Verwendung zur Behandlung einer fetalen Wachstumsrestriktion (IUGR) oder zur parenteralen Ernährung von extrem Frühgeborenen in der 24. bis 34. Schwangerschaftswoche. Die Zusammensetzung umfasst eine Mischung aus Aminosäuren, bestehend aus Isoleucin, Leucin, Lysin, Methionin, Phenylalanin, Threonin, Tryptophan, Valin, Arginin, Histidin, Ornithin, Glycin, Alanin, Prolin, Aspartat, Asparagin, Glutamat, Glutamin, Serin, Tyrosin, Taurin, Hydroxyprolin, Citrullin, Cystein und ist dadurch gekennzeichnet, dass die in der Mischung enthaltenen Aminosäuren in Konzentrationen oder Konzentrationsverhältnissen vorliegen, die den physiologischen Aminosäurenkonzentrationen oder Konzentrationsverhältnissen im Blutplasma eines gesunden Fötus in utero in der 24. bis 34. Schwangerschaftswoche entsprechen und innerhalb folgender Konzentrationsbereiche in der Zusammensetzung vorhanden sind, wobei die Gesamtmenge der in der Mischung enthaltenen Aminosäuren 100 Gew.-% beträgt:

| | | |
|---|---|---|
| Isoleucin | 1,35 - 7,00 | Gew.-% |
| Leucin | 2,43 - 8,00 | Gew.-% |
| Lysin | 7,51 - 15,32 | Gew.-% |
| Methionin | 0,69 - 1,42 | Gew.-% |
| Cystein | 0,1 - 3,00 | Gew.-% |
| Phenylalanin | 1,63 - 3,33 | Gew.-% |
| Threonin | 3,00 - 11,62 | Gew.-% |
| Tryptophan | 1,00 - 2,93 | Gew.-% |
| Valin | 4,37 - 8,91 | Gew.-% |
| Arginin | 2,01 - 9,00 | Gew.-% |
| Histidin | 2,47 - 5,04 | Gew.-% |
| Ornithin | 1,98 - 5,50 | Gew.-% |
| Glycin | 3,77 - 7,69 | Gew.-% |
| Alanin | 5,86 - 11,95 | Gew.-% |
| Prolin | 3,19 - 8,00 | Gew.-% |
| Aspartat | 0,85 - 3,00 | Gew.-% |
| Asparagin | 1,56 - 5,00 | Gew.-% |
| Glutamat | 3,32 - 8,50 | Gew.-% |
| Glutamin | 1,00 - 20,0 | Gew.-% |
| Serin | 2,66 - 5,44 | Gew.-% |
| Tyrosin | 1,00 - 3,40 | Gew.-% |
| Taurin | 0,26 - 6,14 | Gew.-% |
| Hydroxyprolin | 0,10 - 1,25 | Gew.-% |
| Citrullin | 0,10 - 0,74 | Gew.-% |

Bei der Zusammensetzung sind die darin enthaltenen Aminosäuren und deren Konzentrationen an den physiologischen Verhältnissen angepasst, wie sie bei einem gesunden Fötus mit dem gleichen Gewicht in utero vorhanden sind. Dies wird einem Fötus in der 24. bis 34. Schwangerschaftswoche entsprechen.

Die Zusammensetzung umfasst demgemäß eine Mischung aus Aminosäuren oder deren künstliche oder modifizierte Aminosäureanaloge oder Substitute, ausgewählt aus der Gruppe bestehend aus: Isoleucin, Leucin, Lysin, Methionin, Phenylalanin, Threonin, Tryptophan, Valin, Arginin, Histidin, Ornithin, Glycin, Alanin, Prolin, Aspartat, Asparagin, Glutamat, Glutamin, Serin, Tyrosin, Taurin, Hydroxyprolin, Citrullin, Cystein.

Die dem IUGR-betroffenen Fötus oder dem extrem Frühgeborenen zu verabreichenden und in der Zusammensetzung enthaltenen Aminosäuren liegen in Konzentrationen oder Konzentrationsverhältnissen vor, die den physiologischen Aminosäurenkonzentrationen oder Konzentrationsverhältnissen im Blutplasma eines gesunden Fötus in der 24. bis 34. Schwangerschaftswoche in utero entsprechen oder möglichst nahe kommen. Die Aminosäurenkonzentrationen oder Konzentrationsverhältnisse im Blutplasma des Fötus in utero sind über eine Entnahme von Nabelschnurblut ermittelbar.

### Beschreibung der Erfindung:

Kerngedanke der vorliegenden Erfindung ist es, eine Aminosäurezusammensetzung bereitzustellen, deren Aminosäurezusammensetzung und Aminosäurekonzentrationen denen eines gesunden Fötus in utero entspricht, der in etwas das gleiche Körpergewicht aufweist wie der extrem Frühgeborene oder der von IUGR-betroffene Fötus.

Unter die Erfindung fallen nicht nur natürliche sondern auch künstliche oder modifizierte Aminosäuren, oder Substitute solcher Aminosäuren wie z.B. chemisch modifizierte oder künstliche Aminosäuren, Analoge, Homologe oder Derivate davon. Vorzugsweise liegen die Aminosäuren als Gemisch in Lösung vor.

Je nach Anwendungsfall und/oder Zusammensetzung können einzelne oder mehrere Aminosäuren in hochdosierter Form vorliegen und werden bei Anwendung mit Wasser oder einer Glukoselösung auf die gewünschte Verabreichungskonzentration verdünnt.

Die Wahl der einzelnen Aminosäuren in der erfindungsgemäßen Lösung und deren Konzentrationsbereiche bzw. Konzentrationsverhältnisse kann von unterschiedlichen Faktoren abhängen. Zum einen hängt es davon ab, in welcher Schwangerschaftswoche sich der Fötus befindet oder wann die extreme Frühgeburt stattgefunden hat. Zum anderen spielen auch praktische Erwägungen eine Rolle, beispielsweise die Stabilität oder künstliche Synthetisierbarkeit einzelner Aminosäuren. Daher macht es ggf. Sinn, die Zusammensetzung und die Konzentrationen der Aminosäuren in der zu verabreichenden Lösung bedarfsweise an den Entwicklungsstand des Fötus bzw. des Frühgeborenen anzupassen. Gegebenenfalls kann es notwendig sein, Aminosäuren wie Glutamin, Hydroxyprolin oder Citrullin durch künstliche Analoge oder andere Aminosäuren zu ersetzen, oder aus der Lösung ganz heraus zu lassen. Vorzugsweise wird die Lösung jedoch der physiologischen Aminosäurezusammensetzung und den physiologischen Aminosäurekonzentrationen angepasst.

Eine oder mehrere natürlich vorkommende Aminosäuren können durch eine künstliche oder modifizierte Aminosäure ersetzt werden. Demnach umfasst der hier verwendete Begriff "Analog" sowohl künstliche als auch modifizierte Aminosäuren oder Substitute. Solche Analoge oder Substitute natürlicher Aminosäuren sind bekannt.

Die Verabreichungsvolumina und somit die erwünschten Endkonzentrationen der Aminosäurenzusammensetzung bei der Verabreichung an den Fötus bzw. den Frühgeborenen werden gegebenenfalls durch Verdünnung oder Konzentrierung eingestellt.

Die Aminosäurenkonzentration im Blutplasma des Fötus in utero kann über gängige Methoden, beispielsweise durch direkte Entnahme von der Nabelschnur ermittelt werden. Dem Fachmann stehen hierbei Untersuchungsmethoden zur Verfügung, welche für den Fötus wenig risikobehaftet sind. Aus dem Blut des Fötus wird das Blutplasma isoliert und die darin enthaltenen Aminosäuren analysiert. Eine venöse Blutentnahme kann ggf. bei Frühgeborenen vorgenommen werden, um die Aminosäurenzusammensetzung und deren Konzentrationen im Blutplasma zu ermitteln.

IUGR-Föten oder extrem Frühgeborene werden in der Regel im Vergleich zu gesunden Föten pathologische Aminosäureverhältnisse aufweisen, d.h. die Konzentrationen der Aminosäuren und ggf. deren Zusammensetzung wird sich von den normalen physiologischen Verhältnissen unterscheiden. Ziel der Erfindung ist es eine in utero Situation bei gesunden Föten zu modulieren.

Noch unproblematischer ist die Bestimmung der Aminosäurenkonzentration im Blutplasma der Mutter, da hierbei eine einfache venöse Abnahme und anschließende Bestimmung der Aminosäuren erfolgen kann.

Im Prinzip ist es möglich, für jedes Mutter/Fötus-Paar die ideale Aminosäurenkonzentration experimentell zu ermitteln. Es hat sich jedoch herausgestellt, dass innerhalb der in dieser Anmeldung angegebenen Konzentrationsbereiche der Aminosäuren die erwünschten Erfolge erzielt werden. Insbesondere trifft dies für Lösungen zu, deren Aminosäurenzusammensetzung und Konzentrationen denen eines gesunden Fötus in utero in der 24. bis 34. Schwangerschaftswoche entsprechen.

Interessanterweise weist der intrauterine Fötus in seinem Nabelschnurblut eine höhere Aminosäurenkonzentration auf als die entsprechenden Aminosäuren im maternalen Blutplasma. Es wird durch den aktiven Aminosäurentransport durch die Placenta erklärt. Auf Grundlage der physiologischen Aminosäurenkonzentration des Fötus wurden die erfindungsgemäße Aminosäurezusammensetzung entwickelt und die Konzentrationen entsprechend angepasst. Der Behandlungserfolg kann auf diese Weise bei IUGR signifikant gesteigert, eine Wachstumsretardation könnte vermindert, die Schwangerschaft verlängert werden. Die Verwendung einer physiologischen Zusammensetzung mit einer Auswahl der oben aufgeführten Aminosäuren und einem Energieträger wie Glukose soll die Komplikationen einer IUGR oder die Symptome extrem Frühgeborener verhindern oder zumindest vermindern. Gleiches trifft auch zu für die parenterale Ernährung von extrem frühgeborenen aber der 23. Schwangerschaftswoche, bevorzugt in der 24. bis 34. Schwangerschaftswoche, bevorzugt in der 24. bis 30. SSW, besonders bevorzugt in der 24. bis 28. SSW.

### Beschreibung von bevorzugten Ausführungsformen der Erfindung:

In der Tabelle 1 unten sind die Konzentrationsverhältnisse der einzelnen Aminosäuren beim Fötus, der Mutter sowie einer bevorzugten Ausführungsform der erfindungsgemäßen Zusammensetzung zusammengefasst. Ferner sind die Aminosäurenkonzentrationsverhältnisse der Aminosäuren im Nabelschnurblut des Fötus im Vergleich zum Blutplasma der Mutter gezeigt.

**Tabelle 1**

| Bezeichnung | Fötus mg/l | Mutter mg/l | Fötus µmol/l | Mutter µmol/l | Verhältnis Fötus/Mutter | **Erfindung** g/l | **Erfindung Gew.-%** |
|---|---|---|---|---|---|---|---|
| Isoleucin | 9,07 | 6,00 | 69,11 | 45,72 | 1.51 | **4,53** | **1,93** |
| Leucin | 16,35 | 11,28 | 124,64 | 86,00 | 1,45 | **8,17** | **3,48** |
| Lysin | 53,15 | 22,13 | 363,59 | 151,39 | 2,40 | **26,58** | **10,73** |
| Methionin | 4,93 | 2,69 | 33,04 | 18,05 | 1,83 | **2,46** | **0,99** |
| Cystein | 5,00 | | 33,0 | | | | **0,99** |
| Phenylalanin | 12,20 | 8,32 | 73,83 | 50,35 | 1,47 | **6,10** | **2,33** |
| Threonin | 36,43 | 21,00 | 305,85 | 176,33 | 1,73 | **18,22** | **8,14** |
| Tryptophan | 11,72 | 6,74 | 57,37 | 33,00 | 1,74 | **5,86** | **2,05** |
| Valin | 27,70 | 18,20 | 236,48 | 155,37 | 1,52 | **13,85** | **6,24** |
| Arginin | 15,35 | 8,45 | 88,11 | 48,49 | 1,82 | **7,67** | **2,86** |
| Histidin | 17,98 | 14,07 | 115,89 | 90,66 | 1,28 | **8,99** | **3,53** |
| Ornithin | 13,35 | 5,88 | 100,97 | 44,46 | 2,27 | **6,67** | **2,83** |
| Glycin | 18,48 | 11,68 | 246,23 | 155,56 | 1,58 | **9,24** | **5,39** |
| Alanin | 31,88 | 24,06 | 357,79 | 270,03 | 1,33 | **15,94** | **8,36** |
| Prolin | 20,06 | 13,29 | 174,25 | 115,41 | 1,51 | **10,03** | **4,56** |
| Aspartat | 5,73 | 2,56 | 43,04 | 19,25 | 2,24 | **2,86** | **1,21** |
| Asparagin | 10,52 | 3,50 | 79,07 | 26,28 | 3,01 | **5,26** | **2,22** |
| Glutamat | 23,61 | 19,77 | 160,44 | 134,38 | 1,19 | **11,80** | **4,75** |
| Glutamin | 77,01 | 64,63 | 526,90 | 442,21 | 1,19 | **38,50** | **15,54** |
| Serin | 15,94 | 11,13 | 151,70 | 105,87 | 1,43 | **7,97** | **3,81** |
| Tyrosin | 12,97 | 7,36 | 71,56 | 40,64 | 1,76 | **6,48** | **2,38** |
| Taurin | 19,74 | 5,91 | 157,73 | 47,26 | 3,34 | **9,87** | **4,30** |
| Hydroxyprolin | 4,10 | 2,29 | 31,29 | 17,50 | 1,79 | **2,05** | **0,87** |
| Citrullin | 2,80 | 2,80 | 15,96 | 16,00 | 1,00 | **1,40** | **0,52** |

Beachtenswert ist, dass bei dem Aminosäurenkonzentrationsverhältnis zwischen dem Fötus und der Mutter insbesondere die Aminosäuren Asparagin und Taurin signifikant, d.h. um mehr als das 3-fache erhöht sind. Ferner sind auch die Aminosäuren Lysin, Ornithin und Aspartat um mehr als das 2-fache beim Fötus gegenüber der Mutter signifikant erhöht. In einer bevorzugten Ausführungsform ist die erfindungsgemäße Zusammensetzung deshalb dadurch gekennzeichnet, dass die Aminosäurenkonzentration für Asparagin und/oder Taurin dem Verhältnis der Aminosäurenkonzentration im Nabelschnurblut des Fötus zu der Aminosäurenkonzentration im Blutplasma der Mutter für die Aminosäuren Asparagin und/oder Taurin mit wenigstens 1 : 2, vorzugsweise wenigstens 1 : 3 entspricht.

Entsprechend den physiologischen Verhältnissen beim Fötus kann die erfindungsgemäße Zusammensetzung auch anhand der Gewichtsmassen angegeben werden. In der folgenden Tabelle 2 sind die bevorzugten und besonders bevorzugten Konzentrationsbereiche für die einzelnen Aminosäuren für die erfindungsgemäße Aminosäurezusammensetzung angegeben, wobei die Gesamtmenge der Aminosäuren 100 Gew.-% beträgt:

**Tabelle 2**

| Aminosäure | Bevorzugter Konzentrationsbereich | | Besonders bevorzugter Konzentrationsbereich | |
|---|---|---|---|---|
| Isoleucin | 1,35-7,00 | Gew.-% | 1,50 - 3,50 | Gew.-% |
| Leucin | 2,43 - 8,00 | Gew.-% | 2,50 - 5,00 | Gew.-% |
| Lysin | 7,51 -15,32 | Gew.-% | 8,00 - 12,00 | Gew.-% |
| Methionin | 0,69 - 1,42 | Gew.-% | 0,75 - 1,20 | Gew.-% |
| Cystein | 0,1 - 3,00 | Gew.-% | 0,5 - 1,00 | Gew.-% |
| Phenylalanin | 1,63 - 3,33 | Gew.-% | 1,75 - 2,75 | Gew.-% |
| Threonin | 3,00 - 11,62 | Gew.-% | 6,00 - 10,00 | Gew.-% |
| Tryptophan | 1,00 - 2,93 | Gew.-% | 1,50 - 2,50 | Gew.-% |
| Valin | 4,37 - 8,91 | Gew.-% | 5,50 - 7,00 | Gew.-% |
| Arginin | 2,01 - 9,00 | Gew.-% | 2,75 - 3,25 | Gew.-% |
| Histidin | 2,47 - 5,04 | Gew.-% | 3,00 - 4,00 | Gew.-% |
| Ornithin | 1,98 - 5,50 | Gew.-% | 2,55 - 3,50 | Gew.-% |
| Glycin | 3,77 - 7,69 | Gew.-% | 3,77 - 7,69 | Gew.-% |
| Alanin | 5,86 - 11,95 | Gew.-% | 4,50 - 6,50* | Gew.-% |
| Prolin | 3,19 - 8,00 | Gew.-% | 4,00 - 5,50 | Gew.-% |
| Aspartat | 0,85 - 3,00 | Gew.-% | 2,50 - 2,95 | Gew.-% |
| Asparagin | 1,56 - 5,00 | Gew.-% | 1,56 - 5,00 | Gew.-% |
| Glutamat | 3,32 - 8,50 | Gew.-% | 3,50 - 5,00 | Gew.-% |
| Glutamin | 1,00 - 20,00 | Gew.-% | 10,50 - 18,00 | Gew.-% |
| Serin | 2,66 - 5,44 | Gew.-% | 3,00 - 4,50 | Gew.-% |
| Tyrosin | 1,00 - 3,40 | Gew.-% | 1,75 - 3,00 | Gew.-% |
| Taurin | 0,26 - 6,14 | Gew.-% | 3,50 - 5,50 | Gew.-% |
| Hydroxyprolin | 0,10 - 1,25 | Gew.-% | 0,50 - 1,00 | Gew.-% |
| Citrullin | 0,10 - 0,74 | Gew.-% | 0,50 - 0,55 | Gew.-% |

* Zum Teil außerhalb des beanspruchten Bereichs.

In einer bevorzugten Ausführungsform der erfindungsgemäßen Zusammensetzung sind Taurin und/oder Asparagin mit einer Konzentration von wenigstens 5 g/l in der Zusammensetzung enthalten.

In einer bevorzugten Ausführungsform sind die oben erwähnten Aminosäuren durch Analoge oder durch modifizierte Aminosäuren ersetzt. Bestimmte Aminosäuren wie z.B. Glutamin sind in Lösungen recht instabil, so dass diese in peptidischer Form verwendet werden müssen, beispielsweise als Dipeptide oder Polypeptide. Daher sind von der Erfindung als solche Aminosäuren umfasst, welche die oben erwähnten "natürlichen" Aminosäuren ersetzen oder ersetzen können. Diese sind im Stand der Technik hinreichend bekannt.

In einer weiteren bevorzugten Ausführungsform enthält die erfindungsgemäße Zusammensetzung neben den zuvor genannten Aminosäuren oder Aminosäureanalogen zusätzlich Mikroelemente und/oder Spurenelemente. Bevorzugte Mikroelemente oder Spurenelemente sind: Chrom (Cr),Cobalt (Co), Eisen (Fe), Iod (I), Kupfer (Cu), Mangan (Mn), Molybdän (Mo), Selen (Se), Zink (Zn), Fluor (F), Silicium (Si), Arsen (As), Nickel (Ni), Zinn (Sn), Vanadium (V).

In einer weiteren Ausführungsform wird die erfindungsgemäße Aminosäurezusammensetzung in einer Zuckerlösung, vorzugsweise einer 10 %-Glucoselösung vermischt.

Je nach Anwendungsgebiet kann es sinnvoll sein, die Stoffmengen der eingesetzten Aminosäuren in entsprechenden Volumina zu lösen und dem Fötus/Kind zu applizieren, um eine unnötige Volumenbelastung des Herzens zu vermeiden.

In dem nachfolgenden Beispiel ist eine bevorzugte Ausführungsform der erfindungsgemäßen Zusammensetzung dargestellt.

### Beispiel 1

Ein von IUGR (fetaler Wachstumsretardation) betroffener Fötus wird mit einer erfindungsgemäßen Zusammensetzung über ein intravaskuläres perinatales Port-System versorgt (M. Tchirikov et al., Treatment of growth-restricted human fetuses with amino acids and glucose supplementation through a chronic fetal intravascular preinatal port system, Case Report Eur Surg Res 2010;45:45-49). Die Zusammensetzung enthielt die folgenden Aminosäuren in den angegebenen Konzentrationen:

| | |
|---|---|
| Isoleucin | 4,53 g/l |
| Leucin | 8,17 g/l |
| Lysin | 26,58 g/l |
| Methionin | 2,46 g/l |
| Phenylalanin | 6,10 g/l |
| Threonin | 18,22 g/l |
| Tryptophan | 5,86 g/l |
| Valin | 13,85 g/l |
| Arginin | 7,67 g/l |
| Histidin | 8,99 g/l |
| Ornithin | 6,67 g/l |
| Glycin | 9,24 g/l |
| Alanin | 15,94 g/l |
| Prolin | 10,03 g/l |
| Aspartat | 2,86 g/l |
| Asparagin | 5,26 g/l |
| Glutamat | 11,80 g/l |
| Glutamin | 38,50 g/l |
| Serin | 7,97 g/l |
| Tyrosin | 6,48 g/l |
| Taurin | 9,87 g/l |
| Hydroxyprolin | 2,05 g/l |
| Citrullin | 1,40 g/l |

Die Gabe erfolgte zusammen mit einer 10 %-Glucoselösung. Das Port-System ermöglicht eine permanente Versorgung des IUGR-Fötus mit Aminosäuren über die Nabelschnur.

Die Behandlung des Fötus erfolgte über tägliche Infusionen mit einer Aminosäurelösung (5 ml/h; 25 ml/Tag) plus einer 10 %-Glucoselösung (25 ml/Tag).

### Beispiel 2

Ein in der 25. Schwangerschaftswoche extrem früh geborenes Kind wird mit einer erfindungsgemäßen Zusammensetzung intravenös behandelt. Die Zusammensetzung enthält die folgenden Aminosäuren in den angegebenen Konzentrationen:

| | |
|---|---|
| Isoleucin | 1,93 Gew.-% |
| Leucin | 3,48 Gew.-% |
| Lysin | 10,73 Gew.-% |
| Methionin | 0,99 Gew.-% |
| Cystein | 0,99 Gew.-% |
| Phenylalanin | 0,99 Gew.-% |
| Threonin | 2,33 Gew.-% |
| Tryptophan | 8,14 Gew.-% |
| Valin | 2,05 Gew.-% |
| Arginin | 6,24 Gew.-% |
| Histidin | 2,86 Gew.-% |
| Ornithin | 3,53 Gew.-% |
| Glycin | 2,83 Gew.-% |
| Alanin | 5,39 Gew.-% |
| Prolin | 8,36 Gew.-% |
| Aspartat | 4,56 Gew.-% |
| Asparagin | 1,21 Gew.-% |
| Glutamat | 2,22 Gew.-% |
| Glutamin | 4,75 Gew.-% |
| Serin | 15,54 Gew.-% |
| Tyrosin | 3,81 Gew.-% |
| Taurin | 2,38 Gew.-% |
| Hydroxyprolin | 0,87 Gew.-% |
| Citrullin | 0,52 Gew.-% |

## Patentansprüche

1. Zusammensetzung für die Verwendung zur Behandlung einer fetalen Wachstumsrestriktion (IUGR) oder zur parenteralen Ernährung von extrem Frühgeborenen in der 24. bis 34. Schwangerschaftswoche, umfassend eine Mischung aus Aminosäuren, bestehend aus Isoleucin, Leucin, Lysin, Methionin, Phenylalanin, Threonin, Tryptophan, Valin, Arginin, Histidin, Ornithin, Glycin, Alanin, Prolin, Aspartat, Asparagin, Glutamat, Glutamin, Serin, Tyrosin, Taurin, Hydroxyprolin, Citrullin, Cystein,
**dadurch gekennzeichnet, dass** die in der Mischung enthaltenen Aminosäuren in Konzentrationen oder Konzentrationsverhältnissen vorliegen, die den physiologischen Aminosäurenkonzentrationen oder Konzentrationsverhältnissen im Blutplasma eines gesunden Fötus in utero in der 24. bis 34. Schwangerschaftswoche entsprechen und innerhalb folgender Konzentrationsbereiche in der Zusammensetzung vorhanden sind, wobei die Gesamtmenge der in der Mischung enthaltenen Aminosäuren 100 Gew.-% beträgt:
| | | |
|---|---|---|
| Isoleucin | 1,35 - 7,00 | Gew.-% |
| Leucin | 2,43 - 8,00 | Gew.-% |
| Lysin | 7,51 - 15,32 | Gew.-% |
| Methionin | 0,69 - 1,42 | Gew.-% |
| Cystein | 0,1 - 3,00 | Gew.-% |
| Phenylalanin | 1,63 - 3,33 | Gew.-% |
| Threonin | 3,00 - 11,62 | Gew.-% |
| Tryptophan | 1,00 - 2,93 | Gew.-% |
| Valin | 4,37 - 8,91 | Gew.-% |
| Arginin | 2,01 - 9,00 | Gew.-% |
| Histidin | 2,47 - 5,04 | Gew.-% |
| Ornithin | 1,98 - 5,50 | Gew.-% |
| Glycin | 3,77 - 7,69 | Gew.-% |
| Alanin | 5,86 - 11,95 | Gew.-% |
| Prolin | 3,19 - 8,00 | Gew.-% |
| Aspartat | 0,85 - 3,00 | Gew.-% |
| Asparagin | 1,56 - 5,00 | Gew.-% |
| Glutamat | 3,32 - 8,50 | Gew.-% |
| Glutamin | 1,00 - 20,0 | Gew.-% |
| Serin | 2,66 - 5,44 | Gew.-% |
| Tyrosin | 1,00 - 3,40 | Gew.-% |
| Taurin | 0,26 - 6,14 | Gew.-% |
| Hydroxyprolin | 0,10 - 1,25 | Gew.-% |
| Citrullin | 0,10 - 0,74 | Gew.-% |

2. Zusammensetzung zur Verwendung nach Anspruch 1, **dadurch gekennzeichnet, dass** Taurin und/oder Asparagin mit einer Konzentration von wenigstens 5 g/l in der Zusammensetzung enthalten sind.

3. Zusammensetzung zur Verwendung nach einem der Ansprüche 1 oder 2, **dadurch gekennzeichnet, dass** die Aminosäuren in einer 10 %-Glucoselösung vermischt sind.

4. Zusammensetzung zur Verwendung nach einem der Ansprüche 1 bis 3, **dadurch gekennzeichnet, dass** zusätzlich Mikroelemente oder Spurenelemente, vorzugsweise Chrom (Cr),Cobalt (Co), Eisen (Fe), lod (I), Kupfer (Cu), Mangan (Mn), Molybdän (Mo), Selen (Se), Zink (Zn), Fluor (F), Silicium (Si), Arsen (As), Nickel (Ni), Zinn (Sn), Vanadium (V) enthalten sind.

5. Verwendung einer Zusammensetzung nach einem der Ansprüche 1 bis 4 für die Herstellung eines Arzneimittels zur Behandlung einer fetalen Wachstumsrestriktion (IUGR).

6. Verwendung einer Zusammensetzung nach einem der Ansprüche 1 bis 4 für die Herstellung eines Arzneimittels zur parenteralen Ernährung bei extrem Frühgeborenen in der 24. bis 34. Schwangerschaftswoche.

## Claims

1. Composition for use in the treatment of intrauterine growth restriction (IUGR) or in parenteral nutrition for extremely premature infant born in the 24th to 34th week of pregnancy, comprising a mixture of amino acids, consisting of isoleucine, leucine, lysine, methionine, phenylalanine, threonine, tryptophan, valine, arginine, histidine, ornithine, glycine, alanine, proline, aspartate, asparagine, glutamate, glutamine, serine, tyrosine, taurine, hydroxyproline, citrulline and cysteine, **characterised in that** the amino acids contained in the mixture are present in concentrations or concentration ratios corresponding to the physiological amino acid concentrations or concentration ratios in the blood plasma of a healthy foetus in utero in the 24th to 34th week of pregnancy and are present in the composition within the following concentration ranges, the total amount of amino acids contained in the mixture being 100 wt.%:
| | | |
|---|---|---|
| isoleucine | 1.35-7.00 | wt.% |
| leucine | 2.43-8.00 | wt.% |
| lysine | 7.51-15.32 | wt.% |
| methionine | 0.69-1.42 | wt.% |
| cysteine | 0.1-3.00 | wt.% |
| phenylalanine | 1.63-3.33 | wt.% |
| threonine | 3.00-11.62 | wt.% |
| tryptophan | 1.00-2.93 | wt.% |
| valine | 4.37-8.91 | wt.% |
| arginine | 2.01-9.00 | wt.% |
| histidine | 2.47-5.04 | wt.% |
| ornithine | 1.98-5.50 | wt.% |
| glycine | 3.77-7.69 | wt.% |
| alanine | 5.86-11.95 | wt.% |
| proline | 3.19-8.00 | wt.% |
| aspartate | 0.85-3.00 | wt.% |
| asparagine | 1.56-5.00 | wt.% |
| glutamate | 3.32-8.50 | wt.% |
| glutamine | 1.00-20.0 | wt.% |
| serine | 2.66-5.44 | wt.% |
| tyrosine | 1.00-3.40 | wt.% |
| taurine | 0.26-6.14 | wt.% |
| hydroxyproline | 0.10-1.25 | wt.% |
| citrulline | 0.10-0.74 | wt.% |

2. Composition for use according to claim 1, **characterised in that** taurine and/or asparagine are contained in the composition in a concentration of at least 5 g/l.

3. Composition for use according to either claim 1 or claim 2, **characterised in that** the amino acids are mixed in a 10% glucose solution.

4. Composition for use according to any of claims 1 to 3, **characterised in that** microelements or trace elements, preferably chromium (Cr), cobalt (Co), iron (Fe), iodine (I), copper (Cu), manganese (Mn), molybdenum (Mo), selenium (Se), zinc (Zn), fluorine (F), silicon (Si), arsenic (As), nickel (Ni), tin (Sn) and vanadium (V), are additionally contained.

5. Use of a composition according to any of claims 1 to 4 for producing a drug for the treatment of intrauterine growth restriction (IUGR).

6. Use of a composition according to any of claims 1 to 4 for producing a drug for parenteral nutrition in extremely premature infant born in the 24th to 34th week of pregnancy.

## Revendications

1. Composition destinée à être utilisée pour le traitement d'un retard de croissance intra-utérin (RCIU) ou pour l'alimentation parentérale de grands prématurés dans la 24^{e} à la 34^{e} semaine de grossesse, comprenant un mélange d'acides aminés, constitué d'isoleucine, de leucine, de lysine, de méthionine, de phénylalanine, de thréonine, de tryptophane, de valine, d'arginine, d'histidine, d'ornithine, de glycine, d'alanine, de proline, d'aspartate, d'asparagine, de glutamate, de glutamine, de sérine, de tyrosine, de taurine, d'hydroxyproline, de citrulline, de cystéine,
**caractérisée en ce que** les acides aminés contenus dans le mélange sont présents selon des concentrations ou rapports de concentration, qui correspondent à des concentrations d'acides aminés ou à des rapports de concentration physiologiques dans le plasma sanguin d'un fœtus en bonne santé in utero dans la 24^{e} à la 34^{e} semaine de grossesse et sont présents dans la composition selon la plage de concentration suivante, où la quantité totale des acides aminés contenus dans le mélange est de 100 % en poids :
| | |
|---|---|
| Isoleucine | De 1,35 à 7,00 % en poids |
| Leucine | De 2,43 à 8,00 % en poids |
| Lysine | De 7,51 à 15,32 % en poids |
| Méthionine | De 0,69 à 1,42 % en poids |
| Cystéine | De 0,1 à 3,00 % en poids |
| Phénylalanine | De 1,63 à 3,33 % en poids |
| Thréonine | De 3,00 à 11,62 % en poids |
| Tryptophane | De 1,00 à 2,93 % en poids |
| Valine | De 4,37 à 8,91 % en poids |
| Arginine | De 2,01 à 9,00 % en poids |
| Histidine | De 2,47 à 5,04 % en poids |
| Ornithine | De 1,98 à 5,50 % en poids |
| Glycine | De 3,77 à 7,69 % en poids |
| Alanine | De 5,86 à 11,95 % en poids |
| Proline | De 3,19 à 8,00 % en poids |
| Aspartate | De 0,85 à 3,00 % en poids |
| Asparagine | De 1,56 à 5,00 % en poids |
| Glutamate | De 3,32 à 8,50 % en poids |
| Glutamine | De 1,00 à 20,0 % en poids |
| Sérine | De 2,66 à 5,44 % en poids |
| Tyrosine | De 1,00 à 3,40 % en poids |
| Taurine | De 0,26 à 6,14 % en poids |
| Hydroxyproline | De 0,10 à 1,25 % en poids |
| Citrulline | De 0,10 à 0,74 % en poids |

2. Composition destinée à être utilisée selon la revendication 1, **caractérisée en ce que** la taurine et/ou l'asparagine sont présentes selon une concentration d'au moins 5 g/l dans la composition.

3. Composition destinée à être utilisée selon l'une des revendications 1 et 2, **caractérisée en ce que** les acides aminés sont mélangés dans une solution de glucose à 10 %.

4. Composition destinée à être utilisée selon l'une des revendications 1 à 3, **caractérisée en ce que** des oligo-éléments ou micronutriments, de préférence le chrome (Cr), le cobalt (Co), le fer (Fe), l'iode (I), le cuivre (Cu), le manganèse (Mn), le molybdène (Mo), le sélénium (Se), le zinc (Zn), le fluor (F), le silicium (Si), l'arsenic (As), le nickel (Ni), l'étain (Sn), le vanadium (V) sont en outre présents.

5. Utilisation d'une composition selon l'une des revendications 1 à 4, pour la fabrication d'un médicament pour le traitement d'un retard de croissance intra-utérin (RCUI).

6. Utilisation d'une composition selon l'une des revendications 1 à 4, pour la fabrication d'un médicament pour l'alimentation parentérale de grands prématurés dans la 24^{e} à la 34^{e} semaine de grossesse.
